# EUROPÄISCHE PATENTANMELDUNG

(11) **EP 3 747 342 A2**
(43) Veröffentlichungstag der Anmeldung: **09.12.2020**
(21) Anmeldenummer: 20175816.6
(22) Anmeldetag: 20.05.2020
(51) Int. Cl.: A61B 1/00, A61B 1/06, A61B 1/12, A61B 1/313, A61B 1/32, F21V 29/71

(54) **ENDOSKOP, VERFAHREN ZUM BETREIBEN EINES ENDOSKOPS SOWIE VERFAHREN ZUM HERSTELLEN EINES ENDOSKOPS**

(30) Priorität: 03.06.2019 DE 102019003839
(71) Anmelder: Karl Storz SE & Co. KG, 78532 Tuttlingen (DE)
(72) Erfinder: Ulmschneider, Daniel, 78532 Tuttlingen (DE); Czupalla, Christian, 78532 Tuttlingen (DE)
(74) Vertreter: Weidner Stern Jeschke

(57) **Zusammenfassung**

Eine erfindungsgemäßes Endoskop, insbesondere ein Mediastinoskop (1), umfasst einen lang erstreckten Schaft (10) und ein an einem proximalen Endabschnitt (11) des Schafts (10) angeordnetes Kopfteil (30), wobei in dem Kopfteil (30) eine Wärmequelle angeordnet ist, wobei sich innerhalb des Schafts (10) mindestens ein Wärmerohr erstreckt, wobei ein proximaler Endabschnitt des mindestens einen Wärmerohrs mit der Wärmequelle thermisch gekoppelt ist und wobei das Endoskop ein optisches System aufweist, das durch ein in einem distalen Endabschnitt (14) des Schafts (10) angeordnetes Deckglas (27) abgeschlossen ist. Die Wärmequelle ist eine Lichtquelle (32) zur Erzeugung einer Beleuchtungsstrahlung. Das mindestens eine Wärmerohr erstreckt sich in distaler Richtung bis in den distalen Endabschnitt (14) des Schafts (10), und zumindest ein distaler Endabschnitt des Wärmerohrs ist mit dem distalen Endabschnitt (14) des Schafts (10) thermisch gekoppelt ist. Die Erfindung betrifft auch ein Verfahren zum Betreiben eines Endoskops und ein Verfahren zum Herstellen eines Endoskops.

## Beschreibung

Die vorliegende Erfindung betrifft ein Endoskop, insbesondere ein Mediastinoskop, sowie ein Verfahren zum Betreiben eines Endoskops und ein Verfahren zum Herstellen eines entsprechenden Endoskops.

Endoskope für medizinische oder technische Anwendungen weisen einen zur Einführung in einen Hohlraum ausgebildeten lang erstreckten Schaft auf, der optische und/oder elektronische Bauteile zur Aufnahme eines Bildes einer Szene in dem Hohlraum und zur Weiterleitung des aufgenommenen Bildes zu einer außerhalb des Hohlraums angeordneten Anzeige oder Betrachtungseinrichtung enthält. Um die aufzunehmende endoskopische Szene ausreichend zu beleuchten, ist es bekannt, von einer externen Beleuchtungseinrichtung erzeugtes Beleuchtungslicht über ein Lichtleitkabel zu dem Endoskop zu leiten, oder innerhalb des Endoskops eine oder mehrere Lichtquellen, beispielsweise lichtemittierende Dioden (LEDs), anzuordnen. Hierdurch entsteht beim Betrieb jedoch Verlustwärme, die zu einer Erwärmung des Endoskops führt. Ferner kann ein Endoskop weitere Wärmequellen aufweisen, beispielsweise einen elektronischen Bildsensor zur Aufnahme eines endoskopischen Bilds sowie elektronische Schaltungen zur Auswertung der erzeugten Bilddaten.

Derartige Wärmequellen können im distalen (d.h. benutzerfernen) oder im proximalen (d.h. benutzernahen) Endbereich des Endoskops oder auch in einem Handgriff angeordnet sein und dort eine Temperaturerhöhung verursachen. Eine übermäßige Erwärmung auch von Teilbereichen der Oberfläche des Endoskops ist jedoch in der Regel unerwünscht, insbesondere bei Endoskopen für medizinische Anwendungen. So kann eine Erwärmung der Oberfläche des Schafts über eine Temperatur von 41°C hinaus zu einer Schädigung von Körpergewebe führen, mit dem das Endoskop in Kontakt kommt. Ebenso kann eine übermäßige Erwärmung des Handgriffs die Bedienung des Endoskops beeinträchtigen. Es ist daher wünschenswert, die von den Wärmequellen erzeugte Verlustwärme abzuführen bzw. zu verteilen.

In DE 10 2007 032 200 A1 ist ein Endoskop offenbart, das einen Handgriff sowie ein damit verbindbares Sondenteil aufweist, wobei in dem Handgriff ein Beleuchtungssystem mit wenigstens einer Leuchtdiode angeordnet ist. Für die wenigstens eine Leuchtdiode ist ein innerhalb des Handgriffs angeordnetes Trägerelement aus wärmeleitendem Material vorgesehen, mit dem die Leuchtdiode direkt verbunden ist und das mit Gehäuseteilen und/oder Einbauteilen des Handgriffs und des Sondenteils in thermischem Kontakt stehen kann.

Gemäß EP 2 394 567 A1 weist ein Endoskop einen lang erstreckten Schaft, ein Kopfstück an einem proximalen Ende des Schafts, eine in dem Schaft in einem distalen Bereich desselben angeordnete Lichtquelle, die Verlustwärme erzeugt, und eine passive Kühlung auf, die ein in dem Schaft angeordnetes Wärmerohr in Form einer Heatpipe aufweist, die mit der Lichtquelle thermisch gekoppelt ist, um die Verlustwärme in Richtung nach proximal abzuführen. Das Wärmerohr erstreckt sich bis in das Kopfstück des Endoskops, in dem ein Wärmesenkekörper angeordnet ist, mit dem das Wärmerohr thermisch gekoppelt ist und der die Verlustwärme von dem Wärmerohr aufnimmt und direkt oder über ein Gehäuse des Kopfstücks an die Umgebung abgibt.

Aus EP 2 946 718 A1 ist ein Endoskop bekannt, das einen lang erstreckten rohrförmigen Schaft, eine Verlustwärme erzeugende Wärmequelle und eine Heatpipe aufweist, die sich im Inneren des Schafts in Längsrichtung des Schafts erstreckt und mit der Wärmequelle thermisch gekoppelt ist, um von dieser Wärme aufzunehmen und von der Wärmequelle abzuführen. Die Heatpipe ist zwischen ihrem distalen und ihrem proximalen Ende über zumindest eine Teillänge der Heatpipe und über zumindest einen Teilumfang der Heatpipe mit dem Schaft flächig thermisch leitend gekoppelt, um Wärme von der Heatpipe über zumindest eine Teillänge und zumindest einen Teilumfang des Schaftes an die Umgebung abzuführen.

In der DE 10 2014 107 205 A1 ist ein optisches medizinisches Instrument, insbesondere Endoskop oder Exoskop, offenbart, welches einen langerstreckten rohrförmigen Schaft, eine Verlustwärme erzeugende Wärmequelle, und eine Heatpipe aufweist, die sich im Inneren des Schaftes in Längsrichtung des Schaftes erstreckt und ein distales Heatpipeende und ein proximales Heatpipeende aufweist, wobei die Heatpipe mit der Wärmequelle thermisch gekoppelt ist, um von dieser Wärme aufzunehmen und von der Wärmequelle abzuführen.

Die DE 10 2004 045 502 A1 betrifft ein spreizbares medizinisches Instrument für endoskopische Eingriffe mit einem Grundkörper, einem an dem Grundkörper angeordneten Handgriff und mindestens zwei mit dem Handgriff verbundenen Spatelblättern, die über einen Einstellmechanismus zwischen einer geschlossenen Ausgangsstellung und wenigstens einer Arbeitsstellung parallel und/oder winklig gegeneinander verstellbar sind.

Es hat sich jedoch herausgestellt, dass bei den vorgenannten Endoskopen die innerhalb des Endoskops, insbesondere in einem proximalen Endbereich des Endoskops, erzeugte Verlustwärme nicht immer optimal abgeführt bzw. verteilt wird. So kann die bei den vorgenannten Endoskopen erfolgende Abführung und Verteilung der Verlustwärme zu einer Temperaturverteilung der Oberfläche des Endoskops führen, die insbesondere in einer Anfangsphase eines endoskopischen Eingriffs, wenn das Endoskop noch kalt ist, d.h. weitgehend die Umgebungstemperatur hat, nicht optimal ist.

Es ist Aufgabe der vorliegenden Erfindung, ein verbessertes Endoskop anzugeben, insbesondere ein verbessertes Mediastinoskop, wobei das Endoskop bzw. das Mediastinoskop insbesondere hinsichtlich des Wärme-Managements verbessert ist. Weiter ist es Aufgabe der vorliegenden Erfindung, ein entsprechendes Verfahren zum Betreiben eines Endoskops sowie ein Verfahren zum Herstellen eines solchen Endoskops anzugeben.

Diese Aufgabe wird durch ein Endoskop gemäß Anspruch 1 sowie gemäß der unabhängigen Alternativen gemäß Anspruch 5 bzw. 10, durch ein Verfahren zum Betreiben eines Endoskops gemäß Anspruch 12 und durch ein Verfahren zum Herstellen eines Endoskops gemäß Anspruch 13 gelöst.

Vorteilhafte Weiterbildungen der Erfindung ergeben sich aus den Unteransprüchen.

Ein erfindungsgemäßes Endoskop ist vorzugsweise ein medizinisches Endoskop, insbesondere ein Mediastinoskop; das Endoskop kann aber auch beispielsweise ein Laryngoskop oder ein für andere Anwendungen geeignetes Endoskop oder endoskopisches Instrument sein. Vorzugsweise ist das Endoskop ein Video-Endoskop, in besonders bevorzugter Weise ein Video-Mediastinoskop.

Ein erfindungsgemäßes Endoskop weist einen lang erstreckten Schaft auf, der insbesondere zur Einführung in einen körperinneren Hohlraum eines menschlichen oder tierischen Körpers durch eine natürliche oder eine künstlich geschaffene Körperöffnung bemessen ist. Der Schaft ist vorzugsweise starr ausgebildet und kann einen Außenschaft aufweisen, der beispielsweise als näherungsweise zylindrisches Rohr oder auch spatelförmig ausgebildet sein kann. Weiter weist das Endoskop ein an einem proximalen Endabschnitt des Schafts angeordnetes Kopfteil auf. Das Kopfteil kann beispielsweise als Handgriff oder als Teil eines Handgriffs ausgebildet sein, oder es kann zum Befestigen eines oder eines Teils eines Handgriffs ausgebildet sein; das Kopfteil kann andererseits auch ein proximaler Endabschnitt des Schafts sein. Vorzugsweise steht das Kopfteil quer zu einer Längsrichtung des Schafts von dem Schaft ab. In dem Kopfteil ist mindestens eine Wärmequelle angeordnet, welche beim Betrieb des Endoskops Verlustwärme abgibt.

Innerhalb des Schafts erstreckt sich, im Wesentlichen in einer Längsrichtung des Schafts, mindestens ein Wärmerohr, wobei ein proximaler Endabschnitt des mindestens einen Wärmerohrs mit der Wärmequelle thermisch gekoppelt ist. Unter "thermischer Kopplung" wird hier ein direkter, beispielsweise flächiger, Kontakt oder auch eine Verbindung über ein wärmeleitendes Material oder ein oder mehrere Bauteile aus wärmeleitenden Materialien verstanden, wobei der Kontakt bzw. die Verbindung zur Wärmeübertragung geeignet ist. Als wärmeleitendes Material kommen insbesondere metallische Werkstoffe oder auch etwa Wärmeleitpaste oder Wärmeleitkleber in Frage. Im vorliegenden Zusammenhang ist eine solche wärmeleitende Verbindung, die als thermische Kopplung bezeichnet wird, insbesondere eine Verbindung über eine Strecke, die wesentlich kürzer als eine Länge des Schafts oder auch kürzer als ein Durchmesser des Schafts des Endoskops ist. Bei Vorhandensein eines Temperaturgradienten erfolgt somit ein Wärmetransport über die thermische Kopplung. Das Wärmerohr ist insbesondere zur Abführung der Verlustwärme der Wärmequelle mit der Wärmequelle thermisch gekoppelt und zur Weiterleitung zumindest eines Teils der Verlustwärme in distaler Richtung in den Schaft angeordnet. Das Wärmerohr kann auch als "Heatpipe" bezeichnet werden.

Weiter weist das Endoskop ein optisches System zur Aufnahme eines Bilds eines Objektfelds in dem Hohlraum auf, welches objektseitig durch ein Deckglas abgeschlossen ist, das in einem distalen Endabschnitt des Schafts angeordnet ist. Das optische System kann innerhalb des distalen Endabschnitts des Schafts angeordnet sein und beispielsweise ein Objektiv und einen an das Objektiv anschließenden elektronischen Bildsensor umfassen, kann sich aber auch innerhalb des Schafts bis in den proximalen Endabschnitt des Schafts erstrecken. Insbesondere erstreckt sich das optische System parallel zu einer Längsachse des Schafts. Das Deckglas weist vorzugsweise zumindest objektseitig eine ebene Oberfläche auf, die je nach Blickrichtung des Endoskops und je nach Ausgestaltung des Objektivs senkrecht, schräg oder auch parallel zur Längsachse des Schafts stehen kann. Das Deckglas schließt das optische System insbesondere distalseitig ab und kann beispielsweise einen Teil einer distalseitigen Endfläche des Endoskops bilden oder gegenüber einem distalen Ende des Schafts in proximaler Richtung zurückgesetzt sein.

Erfindungsgemäß ist die im Kopfteil des Endoskops angeordnete Wärmequelle mindestens eine Lichtquelle zur Erzeugung einer Beleuchtungsstrahlung, welche zur Beleuchtung des Hohlraums bzw. des Objektfelds in dem Hohlraum etwa durch innerhalb des Schafts verlaufende Lichtleiter bis in den distalen Endabschnitt des Schafts geleitet werden kann. Weiter erfindungsgemäß erstreckt sich das mindestens eine Wärmerohr in distaler Richtung bis in den distalen Endabschnitt des Schafts und somit zumindest näherungsweise bis auf Höhe des Deckglases. Zumindest ein distaler Endabschnitt des Wärmerohrs ist mit dem distalen Endabschnitt des Schafts thermisch gekoppelt. Insbesondere ist das distale Ende oder der distale Endabschnitt des Wärmerohrs mit dem Außenschaft, in welchen das Deckglas eingebettet sein kann oder von welchem das Deckglas zumindest teilweise umschlossen sein kann, thermisch gekoppelt. Aufgrund der thermischen Kopplung erfolgt dabei eine Wärmeübertragung insbesondere quer zur Längsrichtung des Schafts.

Erfindungsgemäß ist erkannt worden, dass die beim Betrieb des Endoskops entstehende Verlustwärme der mindestens einen Lichtquelle genutzt werden kann, um gezielt Oberflächenbereiche des Endoskops zu erwärmen, für die eine höhere Temperatur vorteilhaft ist. Dies gilt insbesondere für Oberflächenbereiche im distalen Endabschnitt des Schafts, etwa für eine Oberfläche des distalen Endabschnitts des Außenschafts und für das Deckglas des optischen Systems. Das erfindungsgemäße Wärme-Management ist somit nicht nur auf die Abführung der Verlustwärme, sondern auf eine gezielte Verteilung und Nutzung zur Erwärmung von Oberflächenbereichen gerichtet.

Dadurch, dass die Wärmequelle eine Lichtquelle zur Erzeugung der Beleuchtungsstrahlung ist, dass sich das Wärmerohr bis in den distalen Endabschnitt des Schafts erstreckt und dass zumindest ein distaler Endabschnitt des Wärmerohrs mit dem distalen Endabschnitt des Schafts thermisch gekoppelt ist, kann die Verlustwärme der Lichtquelle, die beim Betrieb des Endoskops entsteht, dazu genutzt werden, gezielt den distalen Endabschnitt des Schafts des Endoskops zu erwärmen. Hierdurch wird, insbesondere wenn der distale Endabschnitt des Wärmerohrs mit dem Außenschaft thermisch gekoppelt ist, eine gezielte Erwärmung der Oberfläche des distalen Endabschnitts des Schafts des Endoskops ermöglicht. Hierdurch kann es beispielsweise erreicht werden, dass nach der Inbetriebnahme des Endoskops derjenige Abschnitt des Schafts des Endoskops, der zuerst mit dem Körpergewebe eines Patienten in Kontakt kommt, zuerst erwärmt wird und beispielsweise dann, wenn das Endoskop in die Körperöffnung eingeführt wird, bereits eine Oberflächentemperatur nahe an der Körpertemperatur des Patienten hat.

Ferner kann dadurch, dass das Wärmerohr, das zur Ableitung der Verlustwärme mit der mindestens einen Lichtquelle wärmeleitend verbunden ist, in einem solchen Abschnitt des Schafts, in dem das Deckglas des optischen Systems des Endoskops angeordnet ist, mit dem Schaft thermisch gekoppelt ist, erreicht werden, dass das Deckglas, das das optische System objektseitig abschließt, mittels der von der Lichtquelle abgegebenen Verlustwärme zumindest geringfügig erwärmt wird. Dies ist insbesondere in der Anfangsphase eines endoskopischen Eingriffs vorteilhaft, da der Schaft des Endoskops bei der Einführung in den körperinneren Hohlraum häufig deutlich kühler ist als die in dem Hohlraum herrschende Temperatur, so dass die in dem Hohlraum vorhandene Feuchtigkeit sich an dem Deckglas niederschlagen und die endoskopische Sicht beeinträchtigen kann. Auf diese Weise kann somit ein Beschlagen des Deckglases vermieden und die endoskopische Sicht verbessert werden.

Vorzugsweise ist das optische System in einem Optikschaft aufgenommen, der sich in dem Schaft des Endoskops parallel zur Längsachse des Schafts erstreckt, und dessen distaler Endabschnitt mit dem distalen Endabschnitt des Schafts, insbesondere mit dem Außenschaft, thermisch gekoppelt ist. Der Optikschaft kann fest mit dem Schaft des Endoskops verbunden sein, bzw. in diesen eingesetzt sein, und etwa durch Löten oder mit Wärmeleitkleber zumindest abschnittsweise thermisch mit diesem gekoppelt sein. Der Optikschaft kann distalseitig durch das Deckglas abgeschlossen sein. Der Optikschaft kann hermetisch dicht ausgebildet sein, wobei der Optikschaft durch ein näherungsweise zylindrisches Rohr gebildet werden kann, in dessen distales Ende das Deckglas hermetisch dicht eingesetzt ist. In dem Optikschaft kann ferner ein elektronischer Bildsensor aufgenommen sein, der insbesondere ebenfalls im distalen Endabschnitt des Optikschafts angeordnet sein kann, wobei das optische System ein Endoskopobjektiv umfasst, das auf einer Sensorfläche des Bildsensors ein Bild des Objektfelds erzeugt. Dadurch, dass das mindestens eine Wärmerohr bis in den Bereich des Objektivs und des Deckglases reicht, kann auf vorteilhafte Weise eine ausreichende Wärmeübertragung auf den distalen Endabschnitt des Optikschafts und dadurch auf das Deckglas erreicht werden. Die Wärmeübertragung kann dadurch weiter verbessert werden, dass der Optikschaft zumindest in seinem distalen Endabschnitt mit dem Schaft bzw. mit dem Außenschaft thermisch gekoppelt ist, und somit die Wärmeübertragung ebenfalls im Wesentlichen quer zur Längsrichtung des Schafts erfolgt.

In bevorzugter Weise kann es vorgesehen sein, dass das mindestens eine Wärmerohr zusätzlich in einem mittleren Abschnitt, etwa über einen Teil seiner Länge oder über im Wesentlichen seine gesamte innerhalb des Schafts verlaufende Länge, mit dem Schaft thermisch gekoppelt ist. Insbesondere kann das Wärmerohr in seinem mittleren Abschnitt mit dem Außenschaft thermisch gekoppelt sein und zumindest über einen Teil der Länge des Außenschafts, beispielsweise über die Hälfte oder Dreiviertel der Länge des Außenschafts mit dem innerhalb des Schafts verlaufenden mindestens einen Wärmerohr thermisch gekoppelt sein. Der Außenschaft ist vorzugsweise hoch wärmeleitfähig ausgebildet, beispielsweise aus einem metallischen Werkstoff. Auf diese Weise kann ein großer oder überwiegender Teil oder nahezu eine gesamte äußere Oberfläche des Schafts, zur Abführung der von der Lichtquelle erzeugten Verlustwärme genutzt werden, insbesondere kann der Außenschaft als Wärmesenkekörper dienen. Hierdurch kann, zusätzlich zur gezielten Erwärmung des distalen Endabschnitts des Schafts, eine effiziente Wärmeabführung erreicht werden. Ferner kann hierdurch sicherer verhindert werden, dass eine Oberfläche des Schafts, die mit Körpergewebe des Patienten in Berührung kommt, eine maximal zulässige Temperatur überschreitet.

Gemäß einer besonders bevorzugten Ausführungsform der Erfindung weist der Schaft einen Außenschaft auf, der einen überkragenden Abschnitt aufweist, der in distaler Richtung über das Deckglas hinausreicht, wobei sich das mindestens eine Wärmerohr in distaler Richtung nur bis zum Deckglas oder näherungsweise bis zum Deckglas erstreckt. Insbesondere kann es vorgesehen sein, dass sich das mindestens eine Wärmerohr in distaler Richtung nur oder näherungsweise bis auf Höhe des Objektivs oder eines im distalen Endabschnitt des Optikschafts angeordneten elektronischen Bildsensors erstreckt. Der Außenschaft kann beispielsweise als Rohr oder als Spatel ausgebildet sein. Gemäß einer besonders vorteilhaften Ausführungsform ist das Endoskop als Mediastinoskop ausgebildet, wobei der Schaft durch ein längs geschlitztes Rohr gebildet wird, das innenseitig eine sich in Längsrichtung erstreckende Verdickung aufweist, in die der Optikschaft eingebettet ist. Am distalen Ende weist der Schaft einen schräg angeschnittenen, über das distale Ende des Optikschafts in distaler Richtung hinausragenden Abschnitt auf, durch den beim Einsatz des Mediastinoskops ein Arbeitsraum geschaffen wird, innerhalb dessen unter endoskopischer Sicht Manipulationen durchgeführt werden können, beispielsweise die Entnahme einer Biopsie. Dadurch, dass der Außenschaft einen solchen, über das Deckglas distalseitig hinaus ragenden überkragenden Abschnitt aufweist, in den das mindestens eine Wärmerohr nicht hineinreicht, wird eine Ausgestaltung geschaffen, bei der eine Erwärmung des Deckglases derart ermöglicht wird, dass dieses innerhalb des Arbeitsraums den wärmsten Oberflächenbereich darstellt. Hierdurch kann mit besonders hoher Sicherheit vermieden werden, dass das Deckglas beschlägt.

Gemäß einem weiteren Aspekt der Erfindung, der auch unabhängig von den zuvor beschriebenen Ausgestaltungen beansprucht wird, ist das mindestens eine Wärmerohr in eine sich in dem Schaft aus proximaler Richtung längs erstreckende Sacklochbohrung eingesetzt. Die Sacklochbohrung ist insbesondere aus proximaler Richtung in einen Außenschaft des Endoskops eingebracht und distalseitig verschlossen. Gemäß diesem Aspekt der Erfindung ist die Sacklochbohrung weiterhin nahe ihrem distalen Ende durch eine Querbohrung mit einer weiteren, sich in Längsrichtung des Schafts erstreckenden Bohrung des Schafts verbunden, und das mindestens eine Wärmerohr ist in der Sacklochbohrung in Wärmeleitkleber eingebettet. Dadurch, dass das mindestens eine Wärmerohr in eine Sacklochbohrung eingesetzt ist, wodurch eine besonders große Fläche für den Wärmeübergang zur Verfügung steht, und dadurch, dass das mindestens eine Wärmerohr mittels Wärmeleitkleber mit dem Schaft, insbesondere mit dem Außenschaft, thermisch gekoppelt ist, kann auf einfache Weise eine für viele Fälle ausreichende thermische Kopplung zur Übertragung zumindest eines Teils der Verlustwärme der Lichtquelle auf den Schaft bzw. den Außenschaft erreicht werden. Der Wärmeübergang kann weiter verbessert werden, wenn das mindestens eine Wärmerohr mit engen Toleranzen in die Sacklochbohrung eingesetzt ist. Dadurch, dass die Sacklochbohrung nahe ihrem distalen Ende durch eine Querbohrung mit der weiteren Längsbohrung verbunden ist, kann zudem erreicht werden, dass überschüssiger Wärmeleitkleber durch die Querbohrung aus dem distalen Bereich der Sacklochbohrung austreten kann, so dass ein Aufbau eines Gegendrucks, der die weitere Einführung des Wärmerohrs in die Sacklochbohrung verhindern würde, vermieden werden kann. Auf diese Weise kann die Herstellung des Endoskops vereinfacht werden.

Weiterhin ist es bevorzugt, dass die weitere Längsbohrung als eine Durchgangsbohrung des Schafts, insbesondere des Außenschafts, ausgebildet ist, die sich in Längsrichtung des Schafts erstreckt und in der das optische System aufgenommen ist bzw. in die der Optikschaft eingesetzt ist. Hierdurch kann sowohl ein einfacher Aufbau als auch eine einfache Montage und zugleich eine besonders effiziente thermische Kopplung des mindestens einen Wärmerohrs mit dem Schaft erreicht werden.

In besonders vorteilhafter Weise kann es vorgesehen sein, dass die mindestens eine Sacklochbohrung, die mindestens eine Querbohrung und die weitere Längsbohrung in einen Außenschaft des Schafts eingebracht sind, wobei der Außenschaft einstückig ausgebildet ist, vorzugsweise aus einem metallischen Material, beispielsweise aus Edelstahl. Der Außenschaft kann beispielsweise rohr- oder spatelförmig geformt sein. Durch die einstückige Ausgestaltung kann der Wärmeübergang weiter verbessert werden. Ein derartiger einstückiger Außenschaft aus metallischem Material kann beispielsweise durch Laser-Sintern hergestellt werden.

Vorzugsweise weist das Endoskop zwei Wärmerohre auf, die beidseitig des optischen Systems bzw. des Optikschafts angeordnet sind, insbesondere symmetrisch zum Optikschaft, und zumindest abschnittsweise parallel zum optischen System bzw. zum Optikschaft verlaufen. Auf diese Weise kann eine weiter verbesserte Wärmeübertragung auf den Schaft bzw. auf den Außenschaft sowie auf das Deckglas erreichbar sein.

Weiterhin ist es bevorzugt, dass die Lichtquelle durch mindestens eine lichtemittierende Diode (LED) und einen LED-Träger, auf dem die mindestens eine LED befestigt ist, gebildet wird, wobei das mindestens eine Wärmerohr thermisch an den LED-Träger gekoppelt ist. In weiter bevorzugter Weise ist an die mindestens eine LED ein Lichtleiter optisch angekoppelt, der sich durch den Schaft bis zu dessen distalem Endabschnitt erstreckt und zur Weiterleitung der von der Lichtquelle erzeugten Beleuchtungsstrahlung zu einem zu beobachtenden Objektfeld ausgebildet ist. Die mindestens eine LED kann zusammen mit dem proximalen Ende des Lichtleiters mit einer Vergussmasse vergossen sein, um das Eindringen von Feuchtigkeit in die Lichtquelle zumindest weitgehend zu vermeiden. Die mindestens eine LED kann quer zur Längsrichtung des Schafts angeordnet sein.

Gemäß einem weiteren Aspekt der Erfindung, der auch unabhängig von den zuvor beschriebenen Aspekten beansprucht wird, weist das Endoskop einen Handgriff auf, der ein Gehäuse aufweist, wobei in dem Gehäuse eine Elektronikeinheit aufgenommen ist, welche eine Hülle aufweist, die flächig oder formschlüssig an einer Innenseite des Gehäuses anliegt. Der Handgriff kann ganz oder teilweise durch das Kopfteil des Schafts gebildet werden oder mit dem Kopfteil des Schafts verbunden sein, insbesondere kann das Gehäuse des Handgriffs an das Kopfteil angesetzt sein. Der Handgriff kann beispielsweise um 90° zur Längsrichtung des Schafts abgewinkelt sein. Die Elektronikeinheit umfasst insbesondere elektrische und elektronische Schaltungen zur Versorgung und Steuerung der Lichtquelle und/oder zur Versorgung und Steuerung eines elektronischen Bildsensors und/oder zur Bildverarbeitung oder Bildvorverarbeitung der von dem elektronischen Bildsensor gelieferten Bildsignale. Die Hülle der Elektronikeinheit kann hermetisch dicht ausgebildet sein. Durch die Aufnahme der Elektronikeinheit im Handgriff wird vorteilhafterweise eine bessere Raumausfüllung erreicht, sodass eine kompaktere Bauweise des Endoskops ermöglicht wird. Weiterhin wird durch den Handgriff prinzipiell eine Außenfläche des Kopfteils vergrößert, sodass eine schnellere bzw. effizientere Wärmeableitung ermöglicht wird.

Gemäß diesem Aspekt der Erfindung umfasst das Endoskop somit mindestens zwei Wärmequellen, nämlich die Lichtquelle und die Elektronikeinheit, wobei zur Abführung der von der jeweiligen Wärmequelle erzeugten Verlustwärme zwei entsprechende Wärmesenken mit jeweiligen, separaten Wärmepfaden vorgesehen sind. Die von der Elektronikeinheit abgegebene Verlustwärme wird über die flächige Anlage der Hülle der Elektronikeinheit in effizienter Weise an das Gehäuse des Handgriffs und von diesem an die Umgebung abgegeben. Die flächige Anlage der Hülle kann durch entsprechend bearbeitete Wärmeaustauschflächen der Hülle und des Gehäuses gewährleistet werden. Durch Wärmeleitpaste kann eine weitere Verbesserung des Wärmeübergangs erzielt werden.

Die Elektronikeinheit kann insbesondere eine mit elektronischen Schaltungen bestückte Platine sowie einen metallischen Träger umfassen, wobei die Platine und der Träger mittels Wärmeleitpaste oder Wärmeleitkleber thermisch mit der Hülle der Elektronikeinheit gekoppelt sein können, insbesondere kann die Platine mit dem Träger und der Träger mit der Hülle thermisch gekoppelt sein. Hierdurch kann eine weiter verbesserte Abführung der von der Elektronikeinheit erzeugten Verlustwärme ermöglicht werden.

Gemäß einem erfindungsgemäßen Verfahren zum Betreiben eines Endoskops, insbesondere eines Mediastinoskop, wobei das Endoskop einen lang erstreckten Schaft und ein an einem proximalen Endabschnitt des Schafts angeordnetes Kopfteil umfasst, wird eine im Kopfteil angeordnete Lichtquelle in Betrieb genommen, mindestens ein Teil der von der Lichtquelle erzeugten Verlustwärme über mindestens ein Wärmerohr, dessen proximaler Endabschnitt mit der Lichtquelle thermisch gekoppelt ist und das sich innerhalb des Schafts erstreckt, bis in einen distalen Endabschnitt des Schafts geleitet. Hierdurch kann auf einfache Weise die Verlustwärme der Lichtquelle zum Erwärmen des distalen Endabschnitt des Schafts genutzt werden, wobei insbesondere eine Oberfläche des distalen Endabschnitts eines Außenschafts des Endoskops und/oder ein Deckglas, das ein optisches System des Endoskops objektseitig abschließt, erwärmt werden kann. Dies ist insbesondere zu Beginn eines endoskopischen Eingriffs vorteilhaft. Das Endoskop ist insbesondere wie oben beschrieben ausgebildet und ist in besonders bevorzugter Weise ein Video-Mediastinoskop.

In besonders vorteilhafter Weise kann es vorgesehen sein, die Lichtquelle bereits eine Zeitspanne vor der Benutzung des Endoskops in Betrieb zu nehmen, um bereits zu Beginn des Eingriffs eine optimale Temperaturverteilung der Oberfläche des Endoskops zu erreichen.

Gemäß einem erfindungsgemäßen Verfahren zum Herstellen eines Endoskops wird ein Außenschaft des Endoskops bereitgestellt, der mindestens eine aus proximaler Richtung in den Außenschaft hineinreichende Sacklochbohrung, eine weitere Längsbohrung sowie eine Querbohrung aufweist, die die Sacklochbohrung nahe ihrem distalen Ende mit der Längsbohrung verbindet. Die Sacklochbohrung wird zumindest teilweise mit Wärmeleitkleber ausgefüllt. Sodann wird ein Wärmerohr in die Sacklochbohrung aus proximaler Richtung eingesetzt, wobei der Wärmeleitkleber durch die Querbohrung in die Längsbohrung entweicht. Ein Optikschaft wird in die Längsbohrung eingesetzt, wobei überschüssiger Wärmeleitkleber aus der Längsbohrung entfernt werden kann. Das Verfahren kann weitere Schritte umfassen. Das Endoskop kann insbesondere wie oben beschrieben ausgebildet sein.

In besonders bevorzugter Weise ist der Außenschaft, der die mindestens eine Sacklochbohrung, die Querbohrung und die weitere Längsbohrung enthält, einstückig ausgebildet und insbesondere mittels des Lasersinterverfahren hergestellt worden.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
Fig. 1 ein Ausführungsbeispiel eines erfindungsgemäßen Endoskops in einem Längsschnitt;
Fig. 2 eine vergrößerter Längsschnitt des Schafts und des Kopfteils des Endoskops gemäß Fig. 1;
Fig. 3 einen Querschnitt durch den Schaft und das Kopfteil des Endoskops gemäß Fig. 1;
Fig. 4 einen Längsschnitt durch den distalen Endabschnitt des Schafts des Endoskops gemäß Fig. 1 mit horizontaler Schnittebene;
Fig. 5 einen Querschnitt durch den unteren Bereich des Handgriffs des Endoskops gemäß Fig. 1.

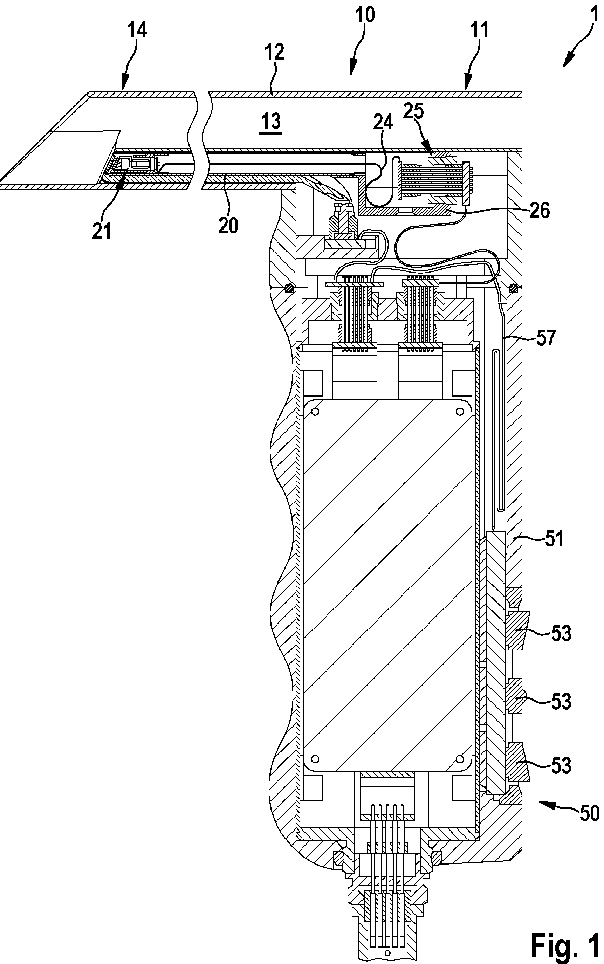

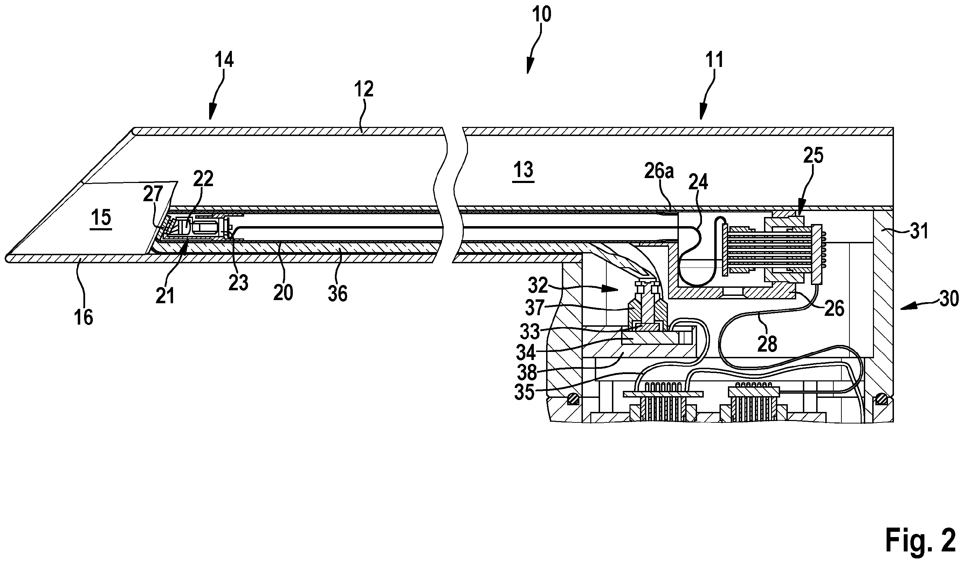

In Fig. 1 ist ein Endoskop gemäß einem Ausführungsbeispiel der vorliegenden Erfindung in einer längsseitigen Schnittansicht sowie in Fig. 2 in einer vergrößerten längsseitigen Schnittansicht des Schafts und des Kopfteils dargestellt, wobei die Schnittebene des Endoskops jeweils vertikal steht und näherungsweise einer Mittelebene des Schafts und des Handgriffs entspricht. Hier und im Folgenden beziehen sich Lage- und Richtungsangaben auf die in Fig. 1 gezeigte Lage des Endoskops. Im dargestellten Ausführungsbeispiel ist das Endoskop ein Mediastinoskop, insbesondere ein Video-Mediastinoskop. Die Erfindung ist jedoch nicht hierauf beschränkt, sondern ein erfindungsgemäßes Endoskop kann in entsprechender Weise beispielsweise als Laryngoskop oder für andere endoskopische Anwendungen ausgebildet sein.

Wie in Fig. 1 gezeigt ist, umfasst das Mediastinoskop 1 einen lang erstreckten Schaft 10, der zur Einführung durch eine Inzision in das Mediastinum ausgebildet ist, sowie einen Handgriff 50. Der Schaft kann insbesondere eine Länge von etwa 10 - 40 cm haben, beispielsweise etwa 20 cm. Der Handgriff 50 ist an einem proximalen Endabschnitt 11 des Schafts 10 angeordnet und steht, bezogen auf die in Fig. 1 gezeigte Lage des Mediastinoskops 1, näherungsweise im rechten Winkel vom Schaft 10 nach unten ab. Der Schaft 10 weist einen Außenschaft auf, der im dargestellten Ausführungsbeispiel durch ein näherungsweise zylindrisches Rohr 12 gebildet wird, das einen in Längsrichtung durchgehenden Schlitz aufweist. Das Rohr 12 stellt den Spatel des Mediastinoskops 1 dar und besteht beispielsweise aus Edelstahl. Das Rohr 12 umschließt einen in Längsrichtung durchgehenden Hohlraum 13, durch den Instrumente beispielsweise zur Entnahme von Biopsien geführt werden können. In dem dem proximalen Endabschnitt 11 entgegengesetzten distalen Endabschnitt 14 des Schafts 10 mündet der Hohlraum 13 in einen Arbeitsraum 15.

Im unteren Bereich des Rohrs 12 ist innenseitig eine Verdickung angeordnet, die eine Längsbohrung aufweist, in die ein Optikschaft 20 eingesetzt ist, der fest mit dem Rohr 12 verbunden ist, beispielsweise durch Löten oder Kleben mit Wärmeleitkleber. Innerhalb des Optikschafts 20 ist eine Bildaufnahmeeinheit 21 aufgenommen, die ein Objektiv 22 und einen elektronischen Bildaufnehmer 23 umfasst. Der elektronische Bildaufnehmer 23 kann beispielsweise ein CCD- oder MOSFET-Sensor sein. Die von dem elektronischen Bildaufnehmer 23 erzeugten Bildsignale werden über eine Flexplatine 24 zu einem elektrischen Steckverbinder 25 am proximalen Ende des Optikschafts 20 geleitet. Weitere Einzelheiten der Anordnung und der elektrischen Verbindungen des elektronischen Bildaufnehmers 23 sind in der am gleichen Tag wie die vorliegende Anmeldung eingereichten deutschen Patentanmeldung desselben Anmelders mit der Bezeichnung "Endoscope and method for manufacturing an endoscope" (unser Zeichen: KST082) beschrieben, welche diesbezüglich durch Bezugnahme in die vorliegende Anmeldung aufgenommen wird.

Der Optikschaft 20 bildet zusammen mit einem proximalseitigen Steckverbindergehäuse 26 einen hermetisch dicht abgeschlossenen Raum. Hierfür ist der Steckverbinder 25 hermetisch dicht in das Steckverbindergehäuse 26 eingesetzt und das distale Ende des Optikschafts durch das ebenfalls hermetisch dicht eingesetzte Deckglas 27 abgeschlossen. Weitere Einzelheiten der hermetisch dichten Ausgestaltung des Optikschafts 20 zusammen mit dem Steckverbindergehäuse 26 sind in der am gleichen Tag wie die vorliegende Anmeldung eingereichten deutschen Patentanmeldung desselben Anmelders mit der Bezeichnung "Video endoscope" (unser Zeichen: KST083) beschrieben, welche diesbezüglich durch Bezugnahme in die vorliegende Anmeldung aufgenommen wird.

Das Rohr 12 des Schafts 10 ist distalseitig schräg angeschnitten und bildet einen über den Optikschaft und über die Position des Deckglases 27 in distaler Richtung hinausragenden, überkragenden Abschnitt 16. Der überkragende Abschnitt 16 des Rohrs 12 umschließt den Arbeitsraum 15, der für chirurgische Manipulationen unter endoskopischer Sicht genutzt werden kann. Hierfür ist das Objektiv 22 für eine zur Längsrichtung des Schafts 10 schräge Blickrichtung ausgebildet und das Deckglas 27 entsprechend schräg gestellt.

Weiter ist in Fig. 1 und Fig. 2 dargestellt, dass an dem proximalen Endabschnitt 11 des Schafts 10 ein Kopfteil 30 angesetzt ist, das ein Kopfteilgehäuse 31 umfasst, in dem das Steckverbindergehäuse 26 sowie eine Lichtquelle 32 aufgenommen sind. Ferner sind in dem Kopfteilgehäuse 31 elektrische Verbindungsleitungen aufgenommen (s.u.). Die Lichtquelle 32 umfasst eine LED 33, die auf einer Trägerplatine 34 aufgesetzt und dort mit elektrischen Leitungen 35 kontaktiert ist. An die LED 33 ist ein Lichtleiter 36 optisch angekoppelt, der durch einen Glasfaserstrang gebildet wird, und der das von der LED 33 erzeugte Beleuchtungslicht bis in den distalen Endabschnitt 14 des Schafts 10 weiterleitet, wo es aus den Glasfasern zur Beleuchtung eines Objektfelds bzw. des Arbeitsraums 15 austritt. Der Glasfaserstrang ist in einer Lichtleiterfassung 37 gehalten. Die Trägerplatine 34 ist auf einem metallischen Träger 38 befestigt, der an der Innenseite des Kopfteilgehäuses 31 montiert ist.

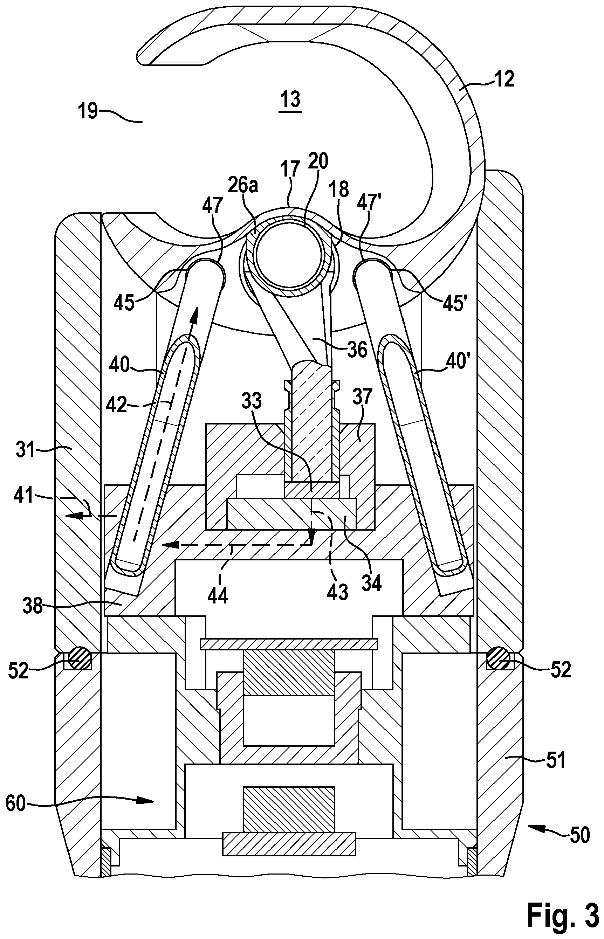

In Fig. 3 ist ein Querschnitt durch den Schaft 10 sowie durch das Kopfteil 30 im Bereich der LED 33 gezeigt, aus proximaler Richtung gesehen. Wie in Fig. 3 dargestellt ist, ist die LED 33 optisch an den Lichtleiter 36 gekoppelt, wobei das proximale Ende des Glasfaserstrangs, der den Lichtleiter 36 bildet, in der Lichtleiterfassung 37 gehalten ist. Der Lichtleiter 36 verläuft unterhalb und seitlich des Optikschafts 20 weiter in distaler Richtung durch das Rohr 12 des Schafts 10. Weiterhin ist in Fig. 3 dargestellt, dass die LED 33 auf der LED-Trägerplatine 34 aufgesetzt ist, welche auf dem Träger 38 befestigt und, etwa durch vollflächiges Aufliegen, wärmeleitend mit diesem verbunden ist. In Bohrungen des Trägers 38 sind zwei Wärmerohre (Heatpipes 40, 40') eingesetzt und mittels Wärmeleitkleber thermisch mit dem Träger 38 gekoppelt. Wie in Fig. 3 durch die Pfeile 41, 42 angedeutet ist, wird die Verlustwärme, die beim Betrieb der LED 33 entsteht und die über die LED-Platine 34 zum Träger 38 geleitet wird, zu einem kleineren Teil direkt in das Kopfteilgehäuse 31 eingeleitet (Pfeil 41) und zum größeren Teil mittels der Heatpipes 40, 40' abgeführt (Pfeil 42). Der Wärmefluss von der LED 33 durch die LED-Platine 34 und innerhalb des metallischen Trägers 38 ist durch die Pfeile 43, 44 angedeutet.

Wie in Fig. 3 ebenfalls gezeigt ist, ist das Rohr 12 des Schafts 10 näherungsweise zylindrisch geformt, jedoch in seinem proximalen Endabschnitt 11 oberseitig abgeflacht. Im unteren Bereich des Rohrs 12 weist dieses eine Verdickung auf, die sich als innenseitiger Wulst 17 in Längsrichtung des Rohrs 12 erstreckt. In dem Wulst 17 ist eine durchgehende mittige Längsbohrung 18 und zwei seitlich parallel zu dieser verlaufende Sacklochbohrungen 45, 45' eingebracht, in denen die Heatpipes 40, 40' geführt sind und jeweils in Wärmeleitkleber 47, 47' eingebettet und thermisch an das Rohr 12 angekoppelt sind. In der Längsbohrung 18 verlaufen der Optikschaft 20 und der Lichtleiter 36. Weiterhin ist in Fig. 3 angedeutet, dass ein Gehäuse 51 des Handgriffs 50 mittels einer Dichtung 52 an das Kopfteilgehäuse 31 angesetzt ist. Innerhalb des Gehäuses 51 ist eine Elektronikeinheit 60 aufgenommen (siehe unten).

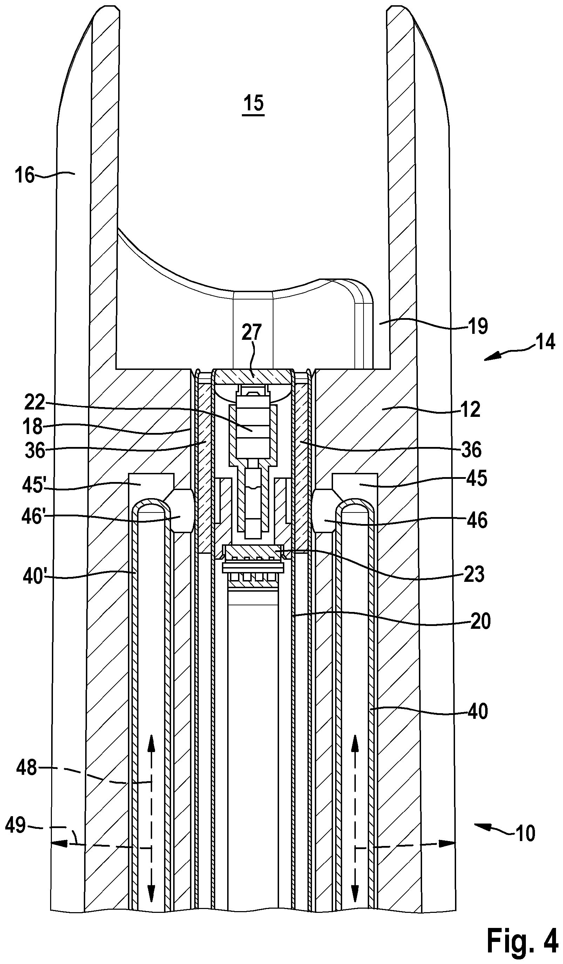

Wie in dem in Fig. 4 gezeigten horizontalen Längsschnitt durch den distalen Endabschnitt 14 des Schafts 10 dargestellt ist, enden die Sacklochbohrungen 45, 45' in distaler Richtung kurz vor dem distalen Ende des Optikrohrs 20. Das Optikrohr 20, in dessen distalem Endabschnitt das Objektiv 22 und der elektronische Bildsensor 23 aufgenommen sind, wird durch das Deckglas 27 hermetisch abgeschlossen. Seitlich des Optikrohrs 20 ist der distale Endabschnitt des Lichtleiters 36 angedeutet. Nahe an dem distalen Ende der Sacklochbohrungen 45, 45' sind Querbohrungen 46, 46' in das Rohr 12 eingebracht, durch die der Wärmeleitkleber 47, 47', in den die Heatpipes 40, 40' eingebettet sind (s. Fig. 3), bei der Montage in die Längsbohrung 18 austreten kann. Der in Fig. 4 gezeigte distale Endabschnitt der Heatpipes 40, 40' ist jeweils mit dem Rohr 12 über den Wärmeleitkleber 47, 47' thermisch gekoppelt. Wie in Fig. 4 ebenfalls gezeigt, bildet der überkragende Abschnitt 16 des Rohrs 12 den Arbeitsraum 15 aus. Weiter ist am distalen Ende des Rohrs 12 der längs durchgehende Schlitz 19 des Rohrs 12 zu erkennen (s. auch Fig. 3).

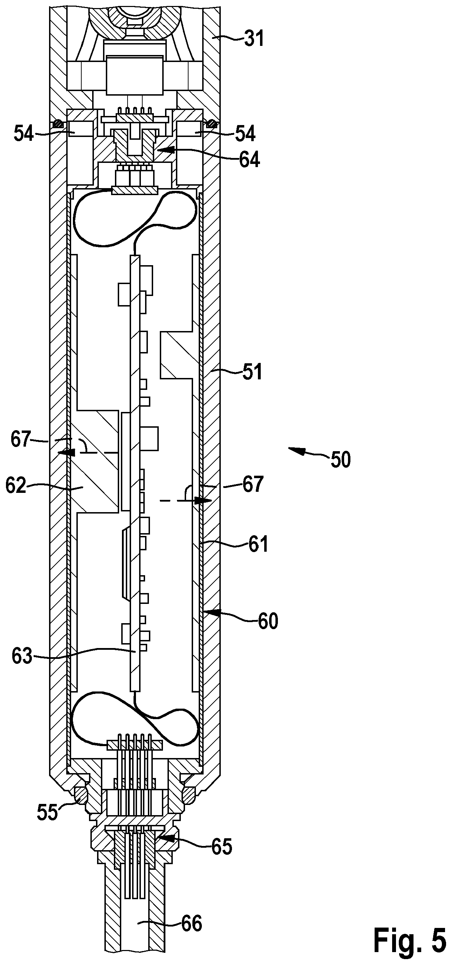

In Fig. 5 ist, näherungsweise in Fortsetzung des in Fig. 3 gezeigten Querschnitts nach unten, der Handgriff 50 im Bereich des Gehäuses 51 aufgeschnitten dargestellt. Innerhalb des Gehäuses 51 des Handgriffs 50 ist die Elektronikeinheit 60 aufgenommen, die eine metallische Hülle 61 aufweist, in der ein Aluminiumträger 62 aufgenommen ist, der die Versorgungsplatine 63 trägt. Die Versorgungsplatine 63 weist elektronische Schaltungen zur Steuerung der LED 33, zur Versorgung des elektronischen Bildsensors 23 und zur Bildvorverarbeitung der von dem elektronischen Bildsensor 23 gelieferten Signale auf. Hierfür ist die Versorgungsplatine 63 über einen Steckverbinder 64 und die entsprechenden Leitungen 28, 35 mit dem Bildsensor 23 bzw. mit der LED 33 verbunden (siehe Fig. 2). Die Leitungen 28, 35 können beispielsweise als Flexplatine oder als Flachbandkabel ausgebildet sein. Ferner ist die Versorgungsplatine 63 über den Steckverbinder 64 und die Leitungen 57 mit einer Mehrzahl von Tasten 53 verbunden, die in das Gehäuse 51 des Handgriffs 50 eingesetzt sind, und mittels derer verschiedene Funktionen des Mediastinoskops 1 gesteuert werden können, etwa die Helligkeit der Beleuchtung und eine elektronische Zoomfunktion. Weiterhin ist die Versorgungsplatine 63 über einen Steckverbinder 65 mit Anschlussleitungen 66 verbunden, die zum Anschluss an eine externe Versorgungs- und Auswertungseinrichtung dienen, die insbesondere auch einen Monitor sowie weitere Bedienelemente umfassen kann.

Der Aluminiumträger 62 ist der Bestückung der Versorgungsplatine 63 angepasst und weist beispielsweise eine ausreichende Dicke auf, um den Zwischenraum zwischen der Hülle 61 und der Versorgungsplatine 63 in den Bereichen, in denen beim Betrieb der Versorgungsplatine 63 die meiste Verlustwärme entsteht, weitgehend auszufüllen. Die Versorgungsplatine 63 ist ferner mittels Wärmeleitpaste oder -kleber mit dem Aluminiumträger 62 thermisch gekoppelt, und dieser ist wiederum mittels Wärmeleitpaste oder -kleber thermisch mit der Hülle 61 gekoppelt. Auch ein Zwischenraum zwischen der Versorgungsplatine 63 und der Hülle 61 kann mit Wärmeleitpaste oder -kleber ausgefüllt sein. Die Hülle 61 liegt flächig an der Innenseite des Gehäuses 51 an. Zur Verbesserung der thermischen Kopplung der Elektronikeinheit 60 an das Gehäuse 51 kann zusätzlich Wärmeleitpaste verwendet werden. Der Wärmetransport durch den Aluminiumträger 62 bzw. Wärmeleitpaste oder -kleber in das Gehäuse ist in Fig. 5 mit den Pfeilen 67 angedeutet.

Die Elektronikeinheit 60 ist hermetisch dicht ausgebildet, wofür die Steckverbinder 64, 65 hermetisch dicht in die metallische Hülle 61 eingesetzt sind. Wie oben erwähnt, ist auch der Optikschaft 20 zusammen mit dem Steckverbindergehäuse 26 hermetisch dicht ausgebildet. Das Mediastinoskop 1 ist insgesamt abgedichtet, wofür beispielsweise die Dichtung 52 vorgesehen ist. Das Mediastinoskop 1 kann somit leicht gereinigt und sterilisiert werden, ohne zerlegt werden zu müssen, und ist bei entsprechender Ausgestaltung insgesamt autoklavierbar. Da dennoch aufgrund der Dichtungen 52 und der Eigenschaften von Glasfasern ein Eindringen von Feuchtigkeit in den Innenraum des Kopfteilgehäuses 31 nicht vollständig vermieden werden kann, ist die LED 33 einschließlich des an die LED 33 optisch angekoppelten proximalen Endes des Lichtleiters 36 bzw. der Lichtleiterfassung 37 von Vergussmasse umschlossen (in den Figuren nicht dargestellt), um die LED 33 zusätzlich abzudichten und gegen das Eindringen von Feuchtigkeit weitestgehend zu schützen.

Wie in Fig. 5 angedeutet ist, ist die Elektronikeinheit 60 mittels Schrauben 54 an dem Kopfteilgehäuse 31 angeschraubt. Die Schrauben 54 sind jedoch im zusammengebauten Zustand des Mediastinoskops 1 nicht zugänglich. Das Gehäuse 51 des Handgriffs 50 wird durch eine Überwurfmutter 55 an der Elektronikeinheit 60 gehalten. Um, beispielsweise im Fall einer Beschädigung der Elektronikeinheit 60, diese zu ersetzen, wird die Überwurfmutter 55 gelöst, wofür ein Spezialwerkzeug notwendig ist. Das Gehäuse 51 des Handgriffs 50 kann sodann von der Hülle 61 der Elektronikeinheit 60 abgezogen werden, so dass die Schrauben 54 zugänglich werden und gelöst werden können, wobei die Leitungen 57 eine ausreichende Länge haben, so dass der Steckverbinder 64 erst nach dem Abziehen der Hülle gelöst werden muss. Nach Lösen der Steckverbinder 64, 65 kann sodann die Elektronikeinheit 60 entnommen und ausgetauscht werden. Auf diese Weise kann ein Ersatz der Elektronikeinheit 60 ermöglicht werden, ohne zugleich den Schaft 10 mit dem Optikschaft 20 ersetzen zu müssen.

Weitere Einzelheiten der hermetisch dichten Ausgestaltung der Elektronikeinheit 60 sowie der Abdichtung und Zerlegbarkeit des Mediastinoskops 1 sind in der am gleichen Tag wie die vorliegende Anmeldung eingereichten deutschen Patentanmeldung desselben Anmelders mit der Bezeichnung "Video endoscope" (unser Zeichen: KST083) beschrieben, welche diesbezüglich durch Bezugnahme in die vorliegende Anmeldung aufgenommen wird.

Wenn das Mediastinoskop 1 in Betrieb genommen wird, entsteht Verlustwärme in der Versorgungsplatine 63 und in der LED 33. Die Verlustwärme der Versorgungsplatine 63 wird, wie oben beschrieben, über den Aluminiumträger 62 und die Hülle 61 an das Gehäuse 51 des Handgriffs 50 weitergeleitet, wobei der Wärmetransport durch Wärmeleitpaste bzw. Wärmeleitkleber verbessert werden kann. Die Verlustwärme der LED 33 wird über die Trägerplatine 34 und den Träger 38 zum kleineren Teil in das Kopfteilgehäuse 31 eingeleitet und zum größeren Teil von den Heatpipes 40, 40' aufgenommen (s. Fig. 3). Die Heatpipes 40, 40' transportieren den betreffenden Teil der Verlustwärme der LED 33 in distaler Richtung in den Schaft 10 des Mediastinoskop 1 hinein und dort zumindest teilweise bis in den distalen Endabschnitt 14 des Schafts 10 (s. Fig. 4). Der bis dorthin übertragene Anteil der Verlustwärme erwärmt über den Wärmeleitkleber 47, 47', in den die Heatpipes 40, 40'in den Sacklochbohrungen 45, 45' eingebettet sind, das Rohr 12 im Bereich des distalen Endabschnitts 14 des Schafts 10. Da der Optikschaft 20 allseitig von dem Rohr 12 umschlossen ist, wird auch der Optikschaft 20 sowie das Objektiv 22 und das in das distale Ende des Optikschafts 22 eingesetzte Deckglas 27 erwärmt. Bereits eine geringe Erwärmung des Deckglases 27 kann ausreichend sein, um ein Beschlagen des Deckglases 27 beim Einführen des Schafts 10 in einen körperinneren Hohlraum zu verhindern.

Die in das Gehäuse 51 eingeleitete Verlustwärme der Versorgungsplatine 63 sowie der in das Kopfteilgehäuse 31 eingeleitete Teil der Verlustwärme der LED 33 wird im Wesentlichen von dort in die Umgebung abgegeben. Der übrige Teil der Verlustwärme der LED 33 wird durch die Heatpipes 40, 40' in den Schaft 10 eingeleitet und über das Rohr 12 ebenfalls an die Umgebung abgegeben bzw. zur Erwärmung des Deckglases 27 genutzt. Auch der elektronische Bildaufnehmer 23 erzeugt beim Betrieb Verlustwärme, die jedoch in der Regel geringer ist als die von der LED 33 abgegebene Verlustwärme. In Fig. 4 ist mit dem Pfeil 48 symbolisch ein entsprechender Wärmefluss innerhalb der Heatpipe 40' angedeutet, der in der Regel in distaler Richtung gerichtet ist, je nach Temperaturgradient und je nach Wärmeabgabe des elektronischen Bildaufnehmers aber prinzipiell auch in proximaler Richtung gerichtet sein kann. Der Wärmefluss von der Heatpipe 40' zur Oberfläche des Rohrs 12 des Schafts 10 ist mit dem Pfeil 49 angedeutet. Wie in Fig. 4 zu erkennen ist, erfolgt der Wärmetransport innerhalb des Rohrs 12 im Wesentlichen oder zumindest teilweise quer zur Längsrichtung des Rohrs 12.

Durch das erfindungsgemäße Wärmemanagement kann einerseits erreicht werden, dass die Verlustwärme, die zum größten Teil im proximalen Bereich des Mediastinoskops anfällt, optimal über distale und proximale Bereiche der Oberfläche des Mediastinoskops abgeführt wird, wobei aber während des Eingriffs eine wärmebedingte Schädigung von Körpergewebe auf jeden Fall vermieden werden kann. Zugleich kann ein Teil der Verlustwärme zur Erwärmung des distalen Endabschnitts des Mediastinoskops genutzt werden, wodurch auch das Deckglas erwärmt werden kann; hierdurch kann ein Beschlagen des Deckglases, speziell zu Beginn einer OP, vermieden werden. Zugleich ist das Mediastinoskop derart ausgestaltet, dass eine einfache Handhabung, Reinigung und Sterilisation beispielsweise durch Autoklavieren ermöglicht wird.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Mediastinoskop
- 10: Schaft
- 11: Proximaler Endabschnitt
- 12: Rohr
- 13: Hohlraum
- 14: Distaler Endabschnitt
- 15: Arbeitsraum
- 16: Überkragender Abschnitt
- 17: Wulst
- 18: Längsbohrung
- 19: Schlitz
- 20: Optikschaft
- 21: Bildaufnahmeeinheit
- 22: Objektiv
- 23: Bildaufnehmer
- 24: Flexplatine
- 25: Steckverbinder
- 26: Steckverbindergehäuse
- 27: Deckglas
- 28: Leitungen
- 30: Kopfteil
- 31: Kopfteilgehäuse
- 32: Lichtquelle
- 33: LED
- 34: Trägerplatine
- 35: Leitungen
- 36: Lichtleiter
- 37: Lichtleiterfassung
- 38: Träger
- 40, 40': Heatpipe
- 41: Pfeil
- 42: Pfeil
- 43: Pfeil
- 44: Pfeil
- 45, 45': Sacklochbohrung
- 46, 46': Querbohrung
- 47, 47': Wärmeleitkleber
- 48: Pfeil
- 49: Pfeil
- 50: Handgriff
- 51: Gehäuse
- 52: Dichtung
- 53: Taste
- 54: Schraube
- 55: Überwurfmutter
- 57: Leitungen
- 60: Elektronikeinheit
- 61: Hülle
- 62: Aluminiumträger
- 63: Versorgungsplatine
- 64: Steckverbinder
- 65: Steckverbinder
- 66: Anschlussleitungen
- 67: Pfeil

## Patentansprüche

1. Endoskop, insbesondere Mediastinoskop (1), mit einem lang erstreckten Schaft (10) und einem an einem proximalen Endabschnitt (11) des Schafts (10) angeordneten Kopfteil (30), wobei in dem Kopfteil (30) eine Wärmequelle angeordnet ist, wobei sich innerhalb des Schafts (10) mindestens ein Wärmerohr erstreckt, wobei ein proximaler Endabschnitt des mindestens einen Wärmerohrs mit der Wärmequelle thermisch gekoppelt ist und wobei das Endoskop ein optisches System aufweist, das durch ein in einem distalen Endabschnitt (14) des Schafts (10) angeordnetes Deckglas (27) abgeschlossen ist, **dadurch gekennzeichnet, dass** die Wärmequelle eine Lichtquelle (32) zur Erzeugung einer Beleuchtungsstrahlung ist, dass das mindestens eine Wärmerohr sich in distaler Richtung bis in den distalen Endabschnitt (14) des Schafts (10) erstreckt und dass zumindest ein distaler Endabschnitt des Wärmerohrs mit dem distalen Endabschnitt (14) des Schafts (10) thermisch gekoppelt ist.

2. Endoskop nach Anspruch 1, **dadurch gekennzeichnet, dass** das optische System in einem Optikschaft (20) aufgenommen ist, der sich in dem Schaft (10) des Endoskops erstreckt und dessen distaler Endabschnitt thermisch mit dem distalen Endabschnitt (14) des Schafts (10) gekoppelt ist.

3. Endoskop nach Anspruch 1 oder 2, **dadurch gekennzeichnet, dass** das mindestens eine Wärmerohr in einem mittleren Abschnitt thermisch mit dem Schaft (10) gekoppelt ist.

4. Endoskop nach einem der vorhergehenden Ansprüche, **dadurch gekennzeichnet, dass** der Schaft (10) einen Außenschaft aufweist, der einen in distaler Richtung über das Deckglas (27) hinaus ragenden überkragenden Abschnitt (16) aufweist, wobei das mindestens eine Wärmerohr sich in distaler Richtung nur bis zum Deckglas (27) oder näherungsweise bis zum Deckglas (27) erstreckt.

5. Endoskop nach einem der vorhergehenden Ansprüche oder nach dem Oberbegriff von Anspruch 1, **dadurch gekennzeichnet, dass** das mindestens eine Wärmerohr in eine sich in Längsrichtung des Schafts (10) erstreckende Sacklochbohrung (45, 45') des Schafts (10) eingesetzt ist, dass die Sacklochbohrung (45, 45') nahe ihrem distalen Ende durch eine Querbohrung (46, 46') mit einer weiteren Längsbohrung (18) des Schafts (10) verbunden ist, und dass das mindestens eine Wärmerohr in der Sacklochbohrung (45, 45') in Wärmeleitkleber (47, 47') eingebettet ist.

6. Endoskop nach Anspruch 5, **dadurch gekennzeichnet, dass** die weitere Längsbohrung (18) eine Durchgangsbohrung ist, wobei in der Durchgangsbohrung das optische System aufgenommen ist.

7. Endoskop nach Anspruch 5 oder 6, **dadurch gekennzeichnet, dass** der Schaft (10) einen Außenschaft umfasst, der die Sacklochbohrung (45, 45'), die Querbohrung (46, 46') und die weitere Längsbohrung (18) aufweist, wobei der Außenschaft einstückig ausgebildet ist.

8. Endoskop nach einem der vorhergehenden Ansprüche, **dadurch gekennzeichnet, dass** das Endoskop zwei Wärmerohre umfasst, die beidseitig des optischen Systems angeordnet sind.

9. Endoskop nach einem der vorhergehenden Ansprüche, **dadurch gekennzeichnet, dass** die Lichtquelle (32) mindestens eine LED (33) sowie einen LED-Träger (38) umfasst, wobei das mindestens eine Wärmerohr an den LED-Träger (38) thermisch gekoppelt ist.

10. Endoskop nach einem der vorhergehenden Ansprüche oder nach dem Oberbegriff von Anspruch 1, **dadurch gekennzeichnet, dass** das Endoskop einen Handgriff (50) mit einem Gehäuse (51) aufweist, wobei im Gehäuse (51) eine Elektronikeinheit (60) aufgenommen ist, die eine Hülle (61) aufweist, welche flächig oder formschlüssig an einer Innenseite des Gehäuses (51) anliegt.

11. Endoskop nach Anspruch 10, **dadurch gekennzeichnet, dass** die Elektronikeinheit (60) eine Platine und einen metallischen Träger der Platine umfasst, welche mittels Wärmeleitpaste und/oder Wärmeleitkleber thermisch mit der Hülle (61) gekoppelt sind.

12. Verfahren zum Betreiben eines Endoskops, insbesondere eines Mediastinoskops (1), welches Endoskop einen lang erstreckten Schaft (10) und ein an einem proximalen Endabschnitt (11) des Schafts (10) angeordnetes Kopfteil (30) umfasst und wobei das Endoskop ein optisches System aufweist, das durch ein in einem distalen Endabschnitt (14) des Schafts (10) angeordnetes Deckglas (27) abgeschlossen ist, wobei eine im Kopfteil (30) angeordnete Lichtquelle (32) zur Erzeugung einer Beleuchtungsstrahlung in Betrieb genommen wird und mindestens ein Teil der von der Lichtquelle (32) erzeugten Verlustwärme über mindestens ein Wärmerohr, dessen proximaler Endabschnitt mit der Lichtquelle (32) thermisch gekoppelt ist und das sich innerhalb des Schafts (10) erstreckt, bis in den distalen Endabschnitt (14) des Schafts (10) geleitet wird.

13. Verfahren zum Herstellen eines Endoskops, insbesondere eines Mediastinoskops (1), wobei ein Außenschaft des Endoskops bereitgestellt wird, der mindestens eine aus proximaler Richtung in den Außenschaft hineinreichende Sacklochbohrung (45, 45'), eine weitere Längsbohrung (18) sowie eine Querbohrung (46, 46') aufweist, die die Sacklochbohrung (45, 45') nahe ihrem distalen Ende mit der Längsbohrung (18) verbindet, die Sacklochbohrung (45, 45') zumindest teilweise mit Wärmeleitkleber ausgefüllt wird, ein Wärmerohr in die Sacklochbohrung (45, 45') eingesetzt wird, wobei der Wärmeleitkleber durch die Querbohrung (46, 46') in die Längsbohrung (18) entweicht, und ein Optikschaft (20) des Endoskops in die Längsbohrung (18) eingesetzt wird.
